# FASCICULE DE BREVET EUROPEEN

(11) **EP 3 234 596 B1**
(45) Date de publication et mention de la délivrance du brevet: **12.08.2020**
(21) Numéro de dépôt: 15825613.1
(22) Date de dépôt: 09.12.2015
(51) Int. Cl.: G01N 33/49, G01N 21/59, G01N 11/16, G01N 21/82

(54) **PROCÉDÉ ET DISPOSITIF DE DÉTERMINATION DU TEMPS DE COAGULATION D'UN ÉCHANTILLON SANGUIN, ET CUVETTE DE RÉACTION**
VERFAHREN UND VORRICHTUNG ZUR BESTIMMUNG DER KOAGULATIONSZEIT EINER BLUTPROBE UND REAKTIONSGEFÄSS
METHOD AND DEVICE FOR DETERMINING THE COAGULATION TIME OF A BLOOD SAMPLE, AND REACTION VESSEL

(30) Priorité: 15.12.2014 FR 1462423
(43) Date de publication de la demande: 25.10.2017
(73) Titulaire: Diagnostica Stago, 92600 Asnières-sur- Seine (FR)
(72) Inventeur: ROUSSEAU, Alain, 75001 Paris (FR); BRUTT, Norbert, 49100 Angers (FR); GUYON, Bertrand, 21300 Chenove (FR)
(74) Mandataire: Verriest, Philippe
(86) Numéro de dépôt international: PCT/FR2015/053399
(87) Numéro de publication internationale: WO 2016/097536

(56) Documents cités:
- EP-A1- 0 325 874
- EP-A1- 2 508 891
- EP-A1- 2 775 292
- US-A1- 2011 224 292

## Description

La présente invention concerne un procédé et un dispositif de détermination du temps de coagulation d'un échantillon sanguin à analyser, et une cuvette de réaction.

Le document EP 0 325 874 divulgue une cuvette de réaction permettant de déterminer le temps de coagulation d'un échantillon sanguin à analyser. A cet effet, le fond de la cuvette de réaction comprend un chemin de roulement curviligne dont la concavité est dirigée vers le haut, sur lequel une bille ferromagnétique est apte à être placée et entraînée en mouvement.

Le document EP 0 325 874 divulgue en outre un procédé de détermination du temps de coagulation, comprenant les étapes suivantes :
- introduire l'échantillon sanguin à analyser dans la cuvette de réaction,
- placer une bille ferromagnétique sur le chemin de roulement de la cuvette de réaction,
- soumettre la bille ferromagnétique à un champ magnétique de manière à déplacer la bille ferromagnétique le long du chemin de roulement selon mouvement oscillatoire,
- exposer l'échantillon sanguin à analyser à un faisceau lumineux incident configuré pour être sensiblement tangent à la bille ferromagnétique lorsque celle-ci est au point le plus bas du chemin de roulement,
- détecter au moins un faisceau lumineux transmis à travers la cuvette de réaction et issu du faisceau lumineux incident de façon à fournir un signal de mesure représentatif de la variation de l'amplitude et/ou de la fréquence du mouvement de la bille ferromagnétique, et
- déterminer le temps de coagulation de l'échantillon sanguin à analyser à partir du signal de mesure.

Cependant, lorsque la variation de l'amplitude et/ou de la fréquence du mouvement de la bille ferromagnétique ne sont pas dues à une augmentation de la viscosité de l'échantillon sanguin à analyser, mais au contraire à la présence de bulles d'air et/ou d'impuretés dans l'échantillon sanguin, le temps de coagulation déterminé par un tel procédé de détermination est alors incorrect, ce qui nuit à la fiabilité d'un tel procédé de détermination.

La présente invention vise à remédier à cet inconvénient.

Le problème technique à la base de l'invention consiste notamment à fournir un procédé et un dispositif de détermination du temps de coagulation d'un échantillon sanguin à analyser qui permettent de déterminer de manière fiable et économique le temps de coagulation.

A cet effet, la présente invention concerne un procédé de détermination du temps de coagulation d'un échantillon sanguin à analyser, comprenant les étapes suivantes :
- prévoir une cuvette de réaction contenant l'échantillon sanguin à analyser, la cuvette de réaction comprenant un fond délimitant un chemin de roulement concave dont la concavité est dirigée vers le haut,
- placer une bille ferromagnétique sur le chemin de roulement de la cuvette de réaction,
- soumettre la bille ferromagnétique à un champ magnétique de manière à déplacer la bille ferromagnétique le long du chemin de roulement selon mouvement oscillatoire,
- exposer l'échantillon sanguin à analyser à un faisceau lumineux incident configuré pour être au moins en partie occulté par la bille ferromagnétique lors d'au moins une partie de son mouvement oscillatoire le long du chemin de roulement,
- détecter au moins un faisceau lumineux transmis à travers la cuvette de réaction et issu du faisceau lumineux incident de façon à fournir un signal de mesure,
- réaliser un premier traitement du signal de mesure de façon à fournir un premier signal représentatif de la variation d'au moins une grandeur physique représentative du mouvement de la bille ferromagnétique,
- réaliser un deuxième traitement du signal de mesure de façon à fournir un deuxième signal représentatif de la variation d'au moins une propriété optique de l'échantillon sanguin à analyser,
- déterminer une première valeur du temps de coagulation de l'échantillon sanguin à analyser à partir du premier signal, et
- déterminer une deuxième valeur du temps de coagulation de l'échantillon sanguin à analyser à partir du deuxième signal.

Un tel procédé de détermination permet de quantifier le temps de coagulation selon deux méthodes différentes, ce qui permet de sécuriser les résultats obtenus. En effet, en cas d'arrêt prématuré du mouvement de la bille ferromagnétique dû à la présence par exemple d'une bulle d'air ou d'impuretés dans l'échantillon sanguin à analyser, la comparaison des première et deuxième valeurs déterminées permet d'identifier une différence entre les deux valeurs de temps de coagulation déterminées. Un opérateur peut alors tenir compte uniquement de la deuxième valeur déterminée qui est moins influencée par l'arrêt de la bille ferromagnétique, ou alors refaire le test pour s'assurer une mesure correcte du temps de coagulation. Le procédé de détermination selon la présente invention permet de ce fait d'obtenir deux mesures indépendantes du temps de coagulation, et donc de fiabiliser les mesures de temps de coagulation.

Selon un mode de mise en œuvre du procédé de détermination, le signal de mesure fourni est obtenu par échantillonnage d'un signal continu à intervalles réguliers, la durée d'un intervalle étant de préférence inférieure à 15 ms, par exemple de l'ordre de 10 ms ou de 4 ms.

Selon un mode de mise en œuvre du procédé de détermination, le premier traitement du signal de mesure est réalisé de telle sorte que le premier signal fourni correspond à l'écart entre une enveloppe haute et une enveloppe basse du signal de mesure.

Selon un autre mode de réalisation de l'invention, l'unité de traitement est configurée de telle sorte que le premier signal fourni correspond à une moyenne glissante de l'écart entre l'enveloppe haute et l'enveloppe basse du signal de mesure, et plus précisément une moyenne glissante de l'écart entre l'enveloppe haute et l'enveloppe basse du signal de mesure sur un ensemble prédéterminé de valeurs de l'écart entre l'enveloppe haute et l'enveloppe basse du signal de mesure, par exemple douze, correspondant à des instants de mesure ou d'échantillonnage successifs, ou sur un intervalle de glissement prédéterminé. Avantageusement, chaque valeur du premier signal pour un instant de mesure ou d'échantillonnage donné est déterminée comme une moyenne glissante d'un ensemble prédéterminé de valeurs de l'écart entre l'enveloppe haute et l'enveloppe basse du signal de mesure S_{M} correspondant à des instants de mesure ou d'échantillonnage successifs précédant l'instant de mesure ou d'échantillonnage donné respectif. De préférence, chaque valeur du premier signal pour un instant de mesure ou d'échantillonnage donné est déterminée comme une moyenne glissante des dernières valeurs de l'écart entre l'enveloppe haute et l'enveloppe basse du signal de mesure, par exemple des douze dernières valeurs de l'écart entre l'enveloppe haute et l'enveloppe basse du signal de mesure.

Selon un mode de mise en œuvre du procédé de détermination, l'enveloppe haute du signal de mesure est déterminée en reliant les maximums locaux du signal de mesure, et l'enveloppe basse du signal de mesure est déterminée en reliant les minimums locaux du signal de mesure.

Selon un mode de mise en œuvre du procédé de détermination, l'au moins une grandeur physique représentative du mouvement de la bille ferromagnétique est l'amplitude et/ou de la fréquence du mouvement de la bille ferromagnétique.

Selon un mode de mise en œuvre du procédé de détermination, l'étape de détermination de la première valeur du temps de coagulation de l'échantillon sanguin à analyser comprend une étape consistant à fournir un signal de base correspondant à une moyenne glissante du premier signal, par exemple sur un intervalle de glissement prédéterminé ou sur un ensemble de valeurs du premier signal correspondant à des instants de mesure ou d'échantillonnage successifs, la première valeur du temps de coagulation de l'échantillon sanguin à analyser étant déterminée à partir du signal de base.

Selon un mode de mise en œuvre du procédé de détermination, chaque valeur du signal de base pour un instant de mesure ou d'échantillonnage donné est déterminée comme une moyenne glissante d'un ensemble de valeurs du premier signal correspondant à des instants de mesure ou d'échantillonnage successifs compris dans un intervalle de temps dont les bornes sont définies par référence à l'instant de mesure ou d'échantillonnage donné. Par exemple, chaque valeur du signal de base pour un instant de mesure ou d'échantillonnage donné est déterminée comme une moyenne glissante d'un ensemble de valeurs du premier signal correspondant à des instants de mesure ou d'échantillonnage compris dans un intervalle de temps, avantageusement compris entre 8 et 12 secondes et est par exemple d'environ 10 secondes, précédent l'instant de mesure ou d'échantillonnage donné.

Selon un autre mode de mise en œuvre du procédé de détermination, chaque valeur du signal de base pour un instant de mesure ou d'échantillonnage donné est déterminée comme une moyenne glissante d'un ensemble de valeurs incluant la valeur du premier signal à l'instant de mesure ou d'échantillonnage donné respectif et la totalité des valeurs du premier signal correspondant à des instants de mesure ou d'échantillonnage précédant l'instant de mesure ou d'échantillonnage donné respectif.

Selon un mode de mise en œuvre du procédé de détermination, l'étape de détermination de la première valeur du temps de coagulation de l'échantillon sanguin à analyser comprend une étape consistant à déterminer le point d'intersection entre le premier signal et un pourcentage prédéterminé du signal de base, la première valeur du temps de coagulation de l'échantillon sanguin à analyser étant la valeur de temps correspondant au point d'intersection déterminé.

Selon un mode de mise en œuvre du procédé de détermination, le pourcentage prédéterminé du signal de base est comprise entre 30 et 60%.

Selon un mode de mise en œuvre du procédé de détermination, le deuxième traitement du signal de mesure est réalisé de telle sorte que le deuxième signal fourni correspond à une enveloppe haute moyennée du signal de mesure.

Selon un mode de mise en œuvre du procédé de détermination, l'unité de traitement est configurée de telle sorte que le deuxième signal fourni correspond à une moyenne glissante de l'enveloppe haute du signal de mesure, et plus précisément une moyenne glissante de l'enveloppe haute du signal de mesure sur un ensemble prédéterminé de valeurs de l'enveloppe haute, par exemple douze, correspondant à des instants de mesure ou d'échantillonnage successifs, ou sur un intervalle de glissement prédéterminé. Avantageusement, chaque valeur du deuxième signal pour un instant de mesure ou d'échantillonnage donné est déterminée comme une moyenne glissante d'un ensemble prédéterminé de valeurs de l'enveloppe haute correspondant à des instants de mesure ou d'échantillonnage successifs précédant l'instant de mesure ou d'échantillonnage donné respectif. De préférence, chaque valeur du deuxième signal pour un instant de mesure ou d'échantillonnage donné est déterminée comme une moyenne glissante des dernières valeurs de l'enveloppe haute, par exemple des douze dernières valeurs de l'enveloppe haute.

Selon un mode de mise en œuvre du procédé de détermination, l'étape de détermination de la deuxième valeur du temps de coagulation de l'échantillon sanguin à analyser comprend une étape consistant à déterminer la pente maximale du deuxième signal, la deuxième valeur du temps de coagulation de l'échantillon sanguin à analyser étant l'instant correspondant à ladite pente maximale.

Selon un mode de mise en œuvre du procédé de détermination, ce dernier comprend une étape consistant à comparer les première et deuxième valeurs du temps de coagulation déterminées.

Selon un mode de mise en œuvre du procédé de détermination, ce dernier comprend en outre une étape consistant à régler l'intensité lumineuse du faisceau lumineux incident en fonction d'une valeur initiale du signal de mesure.

Selon un mode de mise en œuvre du procédé de détermination, la cuvette de réaction est prévu de telle sorte que le chemin de roulement présente son point le plus bas sensiblement en son centre.

Selon un mode de mise en œuvre du procédé de détermination, la valeur initiale du signal de mesure correspond à une position de la bille ferromagnétique sensiblement au niveau du point le plus bas du chemin de roulement.

Selon un mode de mise en œuvre du procédé de détermination, ce dernier comprend en outre une étape consistant à régler, durant une phase initiale du procédé de détermination, au moins un paramètre représentatif du champ magnétique auquel est soumise la bille ferromagnétique en fonction des valeurs initiales du signal de mesure.

Selon un mode de mise en œuvre du procédé de détermination, l'au moins un paramètre représentatif du champ magnétique auquel est soumise la bille ferromagnétique est réglé en fonction des valeurs initiales du premier signal.

Selon un mode de mise en œuvre du procédé de détermination, l'au moins un paramètre représentatif du champ magnétique est la période et/ou l'intensité du champ magnétique auquel est soumise la bille ferromagnétique.

Selon un mode de mise en œuvre du procédé de détermination, la fréquence d'excitation du champ magnétique est proche de la fréquence propre du mouvement oscillatoire de la bille ferromagnétique.

Selon un mode de mise en œuvre du procédé de détermination, le champ magnétique est généré à l'aide d'un système de génération de champ magnétique décalé transversalement par rapport à la direction générale d'extension du chemin de roulement. Une telle disposition du système de génération de champ magnétique permet de disposer verticalement l'élément d'émission à proximité du fond de la cuvette de réaction de manière à diminuer la distance séparant le faisceau lumineux incident et le fond de la cuvette de réaction, et ce sans être gêné par la présence du système de génération de champ magnétique. Il en résulte encore une diminution de la quantité de réactifs et d'échantillon sanguin à introduire dans la cuvette de réaction pour effectuer chaque test, et donc des coûts associés à chaque test.

Selon un mode de mise en œuvre du procédé de détermination, le système de génération de champ magnétique est disposé au moins en partie en regard d'une paroi de la cuvette de réaction s'étendant sensiblement parallèlement au chemin de roulement, et par exemple en regard d'une paroi longitudinale de la cuvette de réaction.

Selon un mode de mise en œuvre du procédé de détermination, le système de génération de champ magnétique comprend un premier et un deuxième électroaimants disposés respectivement à proximité des extrémités du chemin de roulement. Les premier et deuxième électroaimants sont par exemple disposés d'un même côté du chemin de roulement.

Selon un mode de mise en œuvre du procédé de détermination, le champ magnétique peut être réglé en faisant varier par exemple les écarts et/ou la longueur des impulsions électriques appliquées aux bobines des électroaimants.

Selon un mode de mise en œuvre du procédé de détermination, le dispositif de détermination est configuré de telle sorte que, lorsque la cuvette de réaction est reçue dans le logement de réception et la bille ferromagnétique se trouve au point le plus bas du chemin de roulement, la bille ferromagnétique occulte en partie le faisceau lumineux incident.

Selon un mode de mise en œuvre du procédé de détermination, le faisceau lumineux transmis est détecté à l'aide d'un élément de détection situé sensiblement dans l'axe du faisceau lumineux incident.

Selon un mode de mise en œuvre du procédé de détermination, le faisceau lumineux incident est émis à l'aide d'un élément d'émission. L'élément d'émission et l'élément de détection sont par exemple disposés sensiblement dans l'axe du chemin de roulement.

La présente invention concerne en outre un dispositif de détermination du temps de coagulation d'un échantillon sanguin à analyser, le dispositif de détermination comprenant :
- un logement de réception dans lequel est destinée à être reçue une cuvette de réaction contenant l'échantillon sanguin à analyser et une bille ferromagnétique, la cuvette de réaction délimitant un chemin de roulement concave dont la concavité est dirigée vers le haut et sur lequel est placé la bille ferromagnétique,
- un système de génération de champ magnétique configuré pour générer un champ magnétique apte à déplacer la bille ferromagnétique le long du chemin de roulement selon mouvement oscillatoire lorsque la cuvette de réaction est reçue dans le logement de réception,
- un élément d'émission configuré pour émettre un faisceau lumineux incident en direction de l'échantillon sanguin à analyser lorsque la cuvette de réaction est reçue dans le logement de réception, le faisceau lumineux incident étant configuré pour être au moins en partie occulté par la bille ferromagnétique lors d'au moins une partie de son mouvement le long du chemin de roulement,
- un élément de détection configuré pour détecter au moins un faisceau lumineux transmis à travers la cuvette de réaction et issu du faisceau lumineux incident et pour fournir en sortie un signal de mesure, et
- une unité de traitement configurée pour :
   - réaliser un premier traitement du signal de mesure de façon à fournir un premier signal représentatif de la variation d'au moins une grandeur physique représentative du mouvement de la bille ferromagnétique,
   - réaliser un deuxième traitement du signal de mesure de façon à fournir un deuxième signal représentatif de la variation d'au moins une propriété optique de l'échantillon sanguin à analyser,
   - déterminer une première valeur du temps de coagulation de l'échantillon sanguin à analyser à partir du premier signal, et
   - déterminer une deuxième valeur du temps de coagulation de l'échantillon sanguin à analyser à partir du deuxième signal.

Selon un mode de mise en œuvre du dispositif de détermination, le système de génération de champ magnétique est décalé transversalement par rapport à la direction générale d'extension du chemin de roulement.

Selon un mode de réalisation de l'invention, l'élément de détection est situé sensiblement dans l'axe du faisceau lumineux incident,

Selon un mode de réalisation de l'invention, le chemin de roulement présente son point le plus bas sensiblement en son centre.

Selon un mode de mise en œuvre du dispositif de détermination, la bille ferromagnétique et le chemin de roulement sont configurés de telle sorte que, lorsque la bille ferromagnétique se trouve au point le plus bas du chemin de roulement, la bille ferromagnétique occulte en partie le faisceau lumineux incident.

Selon un mode de réalisation de l'invention, le taux d'occultation du faisceau lumineux incident varie entre une valeur minimale correspondant à une position de la bille ferromagnétique au niveau du point le plus bas du chemin de roulement et une valeur maximale correspondant à une position de la bille ferromagnétique la plus éloignée du point le plus bas du chemin de roulement, la valeur minimal étant comprise par exemple entre 5 et 10%.

Selon un mode de réalisation de l'invention, l'élément de détection est un photodétecteur, tel qu'une photodiode.

Selon un mode de réalisation de l'invention, l'élément d'émission est une diode électroluminescente.

Selon un mode de réalisation de l'invention, le dispositif de détermination comprend une bille ferromagnétique destinée à être placée dans le fond de la cuvette de réaction.

Selon un mode de réalisation de l'invention, le dispositif de détermination comprend un système de chargement configuré pour charger et décharger des cuvettes de réaction dans et hors du logement de réaction. Le système de chargement comporte avantageusement un actionneur linéaire, qui peut par exemple comprendre un moteur électrique pas à pas.

La présente invention concerne en outre une cuvette de réaction adaptée au dispositif de détermination selon l'invention, tel que le dispositif de détermination et la cuvette de réaction permettent la mise en œuvre du procédé selon l'invention, la cuvette de réaction comprenant :
- un réceptacle configuré pour contenir un fluide biologique à analyser, le réceptacle comprenant :
   - une partie inférieure comportant un fond délimitant un chemin de roulement concave dont la concavité est dirigée vers le haut, le chemin de roulement présentant son point le plus bas sensiblement en son centre,
   - une partie supérieure délimitant une ouverture d'introduction,
- des premiers moyens d'accrochage configurés pour accrocher la cuvette de réaction à une première cuvette de réaction adjacente selon une première direction d'accrochage, et
- des deuxième moyens d'accrochage configurés pour accrocher la cuvette de réaction à une deuxième cuvette de réaction adjacente selon une seconde direction d'accrochage qui est sensiblement perpendiculaire à la première direction d'accrochage,
la cuvette de réaction contenant une bille ferromagnétique placée sur le chemin de roulement, le chemin de roulement étant destiné à guider un mouvement oscillatoire de la bille ferromagnétique, la bille ferromagnétique étant configurée pour occulter au moins en partie un faisceau lumineux incident émit par l'élément d'émission du dispositif de détermination lors d'au moins une partie du mouvement de la bille ferromagnétique le long du chemin de roulement et lorsque la cuvette de réaction est reçue dans le logement de réception du dispositif de détermination, la cuvette de réaction étant configurée de telle sorte que l'élément d'émission et l'élément de détection sont disposés sensiblement dans l'axe du chemin de roulement lorsque la cuvette de réaction est reçue dans le logement de réception du dispositif de détermination,
la cuvette de réaction étant caractérisée en ce que la largeur de la partie inférieure du réceptacle transversalement à la direction générale d'extension du chemin de roulement est inférieure la largeur de la partie supérieure du réceptacle transversalement à la direction d'extension du chemin de roulement, et en ce que le chemin de roulement est décalé transversalement par rapport à un plan longitudinal médian de la partie supérieure du réceptacle.

Par conséquent, le chemin de roulement est plus proche d'une première paroi longitudinale de la cuvette de réaction que d'une deuxième paroi longitudinale de la cuvette de réaction opposée à ladite première paroi longitudinale.

Une telle configuration de la cuvette de réaction permet d'effectuer des mesures de temps de coagulation dans un volume réactionnel réduit, par exemple inférieur à 90 µL, et donc de réduire la quantité d'échantillon prélevée et également la quantité de réactifs utilisée. Il en résulte ainsi une diminution importante des coûts associés à chaque test effectué.

En outre, dans le cadre de mesures immunologiques utilisant des particules magnétiques, une telle configuration de la cuvette de réaction assure un positionnement d'un aimant ou électroaimant, lors des opérations de lavage des particules magnétiques, au plus près de la zone réactionnelle de la cuvette de réaction (en le plaçant sensiblement au contact de la paroi longitudinale de la cuvette de réaction la plus proche du chemin de roulement), et donc une attraction magnétique optimale des particules magnétiques contre une paroi longitudinale de la cuvette de réaction, ce qui permet d'éviter tout risque de retrait d'une partie des particules magnétiques liées à l'analyte à quantifier hors de la cuvette de réaction avec la solution de lavage.

De plus, dans le cadre encore de mesures immunologiques utilisant des particules magnétiques, une telle configuration de la cuvette de réaction assure un positionnement d'un élément de lecture optique au plus près de la zone réactionnelle de la cuvette de réaction, et donc des résultats de mesure précis et fiables.

Selon un mode de réalisation de l'invention, la partie supérieure du réceptacle est évasée en direction de l'ouverture d'introduction.

Selon un mode de réalisation de l'invention, la partie inférieure du réceptacle est de forme sensiblement parallélépipédique et est allongée selon la direction générale d'extension du chemin de roulement.

Selon un mode de réalisation de l'invention, les premiers moyens d'accrochage comportent au moins une languette d'accrochage dirigée vers le bas et s'étendant à partir d'un bord supérieur de la partie supérieure du réceptacle.

Selon un mode de réalisation de l'invention, la cuvette de réaction comporte une encoche ménagée sur un bord supérieur de la partie supérieure du réceptacle opposé au bord supérieur à partir duquel s'étend la languette d'accrochage, la languette d'accrochage d'une cuvette de réaction étant destinée à coopérer avec l'encoche d'une cuvette de réaction adjacente selon la première direction d'accrochage.

Selon un mode de réalisation de l'invention, les deuxièmes moyens d'accrochage comportent un premier crochet ouvert vers le haut et un deuxième crochet ouvert vers le bas, le premier crochet ouvert vers le haut étant configuré pour venir en prise avec le deuxième crochet ouvert vers le bas d'une cuvette de réaction adjacente, les premier et deuxième crochets étant ménagés sur une embase de la cuvette de réaction, le long de deux bords opposés et orthogonaux au bord supérieur à partir duquel s'étend la languette d'accrochage.

Selon un mode de réalisation de l'invention, le chemin de roulement présente la forme d'une portion de cylindre de rayon compris entre 8 et 10 mm.

De toute façon l'invention sera bien comprise à l'aide de la description qui suit en référence au dessin schématique annexé représentant, à titre d'exemples non limitatifs, une forme d'exécution de ce dispositif de détermination et de cette cuvette de réaction.
Figure 1 est une vue en perspective d'une cuvette de réaction selon l'invention.
Figure 2 est une vue en coupe transversale, en perspective, de la cuvette de réaction de la figure 1.
Figure 3 est une vue en coupe longitudinale de la cuvette de réaction de la figure 1 équipée d'une bille ferromagnétique.
Figure 4 est une vue en coupe transversale de la cuvette de réaction de la figure 1 équipée d'une bille ferromagnétique.
Figures 5 à 8 sont des vues en perspective d'un dispositif de détermination de temps de coagulation selon l'invention équipé d'une cuvette de réaction et dans différentes positions de fonctionnement.
Figure 9 est une vue schématique en coupe montrant la disposition relative entre un système de génération de champ magnétique du dispositif de détermination de la figure 5 et une cuvette de réaction équipant ce dispositif de détermination.
Figures 10 à 13 sont des vues partielles en coupe longitudinale du dispositif de détermination de la figure 5 montrant différentes positions occupées par une bille ferromagnétique placée sur un chemin de roulement de la cuvette de réaction.
Figure 15 est un diagramme représentant l'évolution de l'amplitude d'un signal de mesure en fonction du temps.
Figure 16 est un diagramme représentant l'évolution de l'amplitude des enveloppes basse et haute du signal de mesure en fonction du temps.
Figure 17 est un diagramme représentant l'évolution de l'écart entre les enveloppes haute et basse du signal de mesure et l'évolution d'un pourcentage prédéterminé d'un signal de base déterminé à partir de l'écart entre les enveloppes haute et basse du signal de mesure.
Figure 18 est un diagramme représentant l'évolution de l'amplitude d'une enveloppe haute moyennée du signal de mesure en fonction du temps.

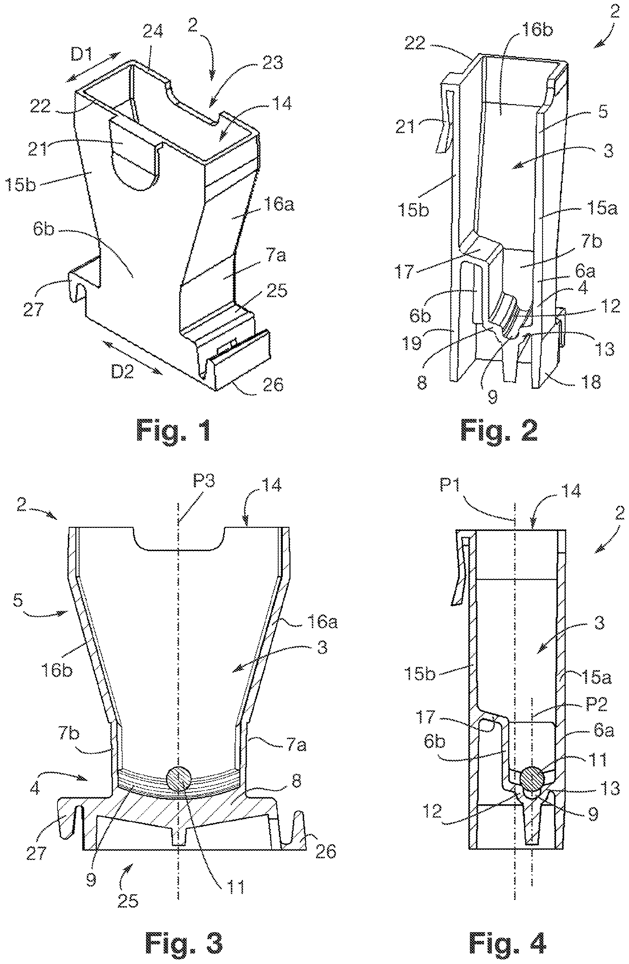

Les figures 1 à 4 représentent une cuvette de réaction 2 unitaire en matière plastique transparente aux faisceaux lumineux. La cuvette de réaction 2 comprend un réceptacle 3 configuré pour contenir un fluide biologique à analyser, tel qu'un échantillon sanguin. Le réceptacle 3 présente par exemple une hauteur de l'ordre de 22 mm et peut par exemple contenir jusqu'à 600 µL de fluide biologique à analyser.

Le réceptacle 3 comprend une partie inférieure 4 et une partie supérieure 5 prolongeant la partie inférieure 4. La partie inférieure 4 est de forme sensiblement parallélépipédique et présente par exemple une longueur de l'ordre de 8 mm et une largeur de l'ordre de 3 mm. La partie inférieure 4 comporte deux parois longitudinales 6a, 6b parallèles entre elles, deux parois transversales 7a, 7b parallèles entre elles et un fond 8. Le fond 8 délimite un chemin de roulement 9 concave dont la concavité est dirigée vers le haut.

Le chemin de roulement 9 est allongé dans le sens longitudinal de la partie inférieure 4 du réceptacle 3 et présente son point le plus bas sensiblement en son centre. Le chemin de roulement 9 est destiné à guider un mouvement oscillatoire d'une bille ferromagnétique 11. Le chemin de roulement 9 peut par exemple être curviligne et être sensiblement en V, ou comme cela est visible sur la figure 3, présenter la forme d'une portion de cylindre. Selon le mode de réalisation représenté sur les figures, le chemin de roulement 9 est délimité par deux rails latéraux 12, 13 ménagés dans le fond 8 de la partie inférieure 4 du réceptacle 3. Ces deux rails latéraux 12, 13 permettent plus particulièrement de guider le mouvement oscillant de la bille ferromagnétique 11 dans la cuvette de réaction 2.

La partie supérieure 5 du réceptacle 3 s'évase à l'opposé du fond 8 et délimite une ouverture d'introduction 14. La partie supérieure 5 présente par exemple une forme générale tronconique. La partie supérieure 5 comporte deux parois longitudinales 15a, 15b parallèles entre elles, et deux parois transversales 16a, 16b reliant les parois longitudinales 15a, 15b entre elles, les parois longitudinales 15a, 15b et les parois transversales 16a, 16b délimitant l'ouverture d'introduction 14.

Selon le mode de réalisation représenté sur les figures, la partie supérieure 5 comporte en outre une paroi de liaison 17 reliant les parois longitudinales 6b, 15b, la paroi de liaison 17 étant inclinée par rapport aux parois longitudinales 6b, 15b. Selon ce mode de réalisation de l'invention, les parois longitudinales 6a, 15a sont coplanaires tandis que les parois longitudinales 6b, 15b sont parallèles et décalées l'une par rapport à l'autre.

On définit la direction transversale D1 comme la direction orthogonale aux parois longitudinales 6a, 6b et la direction longitudinale D2 comme la direction orthogonale aux parois transversales 7a, 7b. On définit également le plan P1 comme le plan longitudinal médian de la partie supérieure 5 du réceptacle 3, le plan P2 comme le plan longitudinal médian de la partie inférieure 4 du réceptacle 3, et le plan P3 comme le plan transversal médian P3 du réceptacle (voir figures 3 et 4).

Comme cela est plus particulièrement visible sur les figures 2 et 4, la largeur de la partie inférieure 4 du réceptacle 3 perpendiculairement à la direction générale d'extension du chemin de roulement 9, c'est-à-dire selon la direction D1, est inférieure la largeur de la partie supérieure 5 du réceptacle 3 perpendiculairement à la direction d'extension du chemin de roulement 9, c'est-à-dire selon la direction D1.

En outre, la partie inférieure 4 du réceptacle 3, et plus particulièrement le chemin de roulement 9, est décalé transversalement par rapport au plan longitudinal médian P1 de la partie supérieure 5 du réceptacle 3. Par conséquent, le chemin de roulement 9 est plus proche de la paroi longitudinale 6a de la partie inférieure 4 que de la paroi longitudinale 6b.

Selon le mode de réalisation représenté sur les figures, la cuvette de réaction 2 comprend en outre une première paroi de finition 18 s'étendant dans le prolongement de la paroi longitudinale 6a à l'opposée de l'ouverture d'introduction 14, et une deuxième paroi de finition 19 s'étendant dans le prolongement de la paroi longitudinale 15b à l'opposée de l'ouverture d'introduction 14.

La cuvette de réaction 2 comprend également une languette d'accrochage 21 dirigée vers le bas et s'étendant à partir d'un bord longitudinal supérieur 22 de la partie supérieure 5 du réceptacle 3. La cuvette de réaction 2 comporte en outre une encoche 23 ménagée sur un bord longitudinal supérieur 24 de la partie supérieure 5 opposé au bord longitudinal supérieur 22. L'encoche 23 est de dimensions adaptées à celles de la languette d'accrochage 21 de telle sorte que la languette d'accrochage 21 d'une cuvette de réaction 2 est destinée à coopérer avec l'encoche 23 d'une cuvette de réaction 2 adjacente selon la direction D1 pour réaliser l'accrochage de deux cuvettes de réaction 2 adjacentes.

En outre, la cuvette de réaction 2 comporte une embase 25 en partie inférieure, dans laquelle sont ménagés, le long de deux bords opposés parallèles à la direction D1, un premier débord 26 formant un premier crochet ouvert vers le haut et un deuxième débord 27 formant un deuxième crochet ouvert vers le bas. Le premier crochet ouvert vers le haut est configuré pour venir en prise avec le deuxième crochet ouvert vers le bas d'une cuvette de réaction 2 adjacente selon la direction D2, pour réaliser l'accrochage de deux cuvettes de réaction 2 adjacentes.

Grâce à la structure de la languette d'accrochage 21 et des premier et deuxièmes débords 26, 27, il est possible d'accrocher des cuvettes de réaction 2 les unes aux autres selon deux directions orthogonales, de façon manuelle ou automatique, pour former des plaques. En outre, les débords 26, 27 permettent d'avoir des dimensions hors tout des cuvettes de réaction 2 qui soient sensiblement les mêmes dans leurs parties supérieures 5 et dans leurs parties inférieures 4 de telle sorte qu'assemblées entre elles, les cuvettes de réaction 2 constituent une plaque plane. Ceci permet d'ordonner les cuvettes de réaction 2 pour les stocker de manière simple, compacte, tout en permettant de détacher aisément une cuvette de réaction 2 de la plaque correspondante.

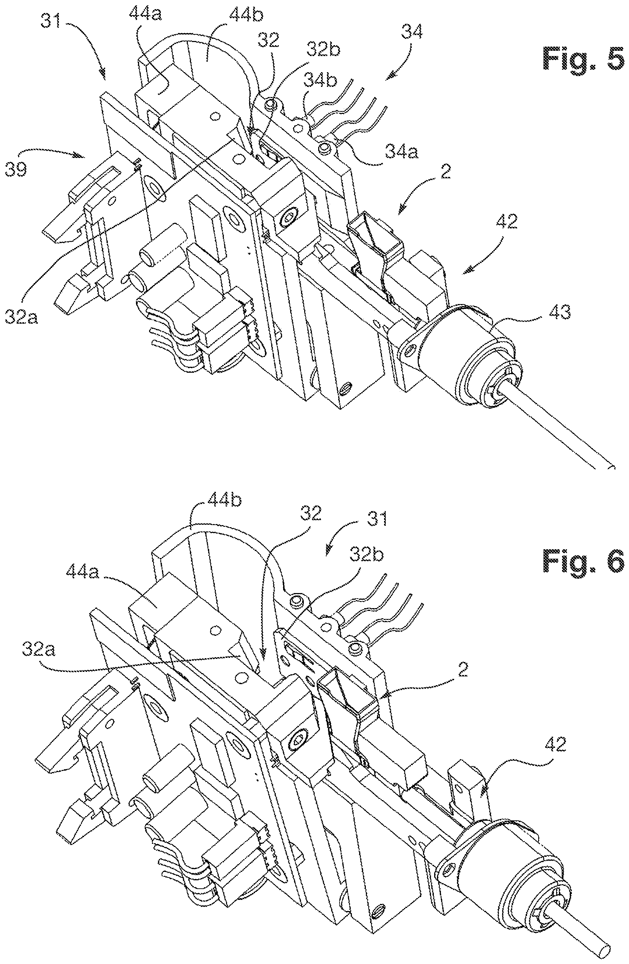

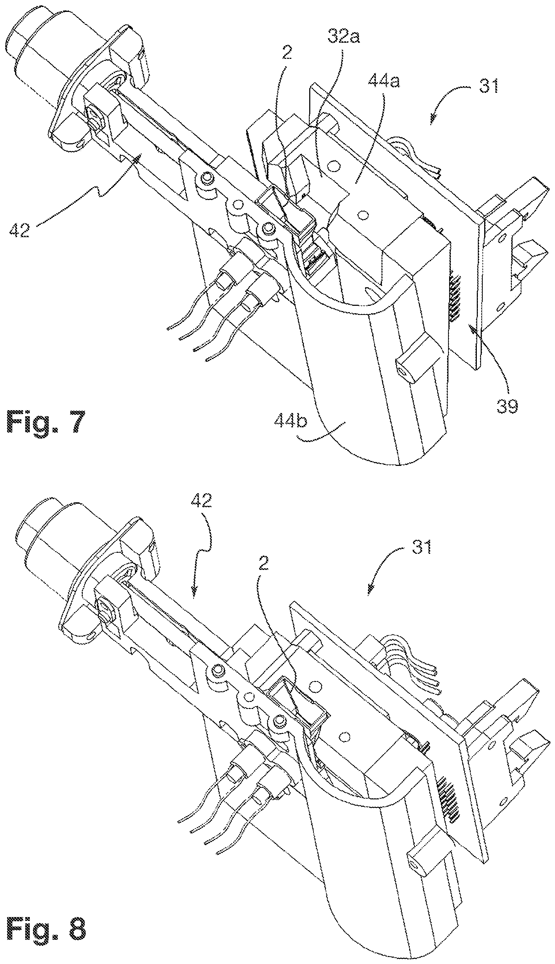

Les figures 5 à 13 représentent un dispositif de détermination 31 configuré pour déterminer le temps de coagulation d'un échantillon sanguin à analyser.

Le dispositif de détermination 31 comprend un logement de réception 32 dans lequel est destinée à être reçue une cuvette de réaction 2 contenant l'échantillon sanguin à analyser 33 et une bille ferromagnétique 11 placée sur le chemin de roulement 9.

Le dispositif de détermination 31 comprend également un système de génération de champ magnétique 34 configuré pour générer un champ magnétique apte à déplacer la bille ferromagnétique 11 le long du chemin de roulement 9 selon mouvement oscillatoire lorsque la cuvette de réaction 2 est reçue dans le logement de réception 32. La fréquence d'excitation du champ magnétique généré par le système de génération de champ magnétique 34 est avantageusement proche de la fréquence propre du mouvement oscillatoire de la bille ferromagnétique 11, et est par exemple de l'ordre de 3,125 Hz (période de l'ordre de 320 ms).

Le dispositif de détermination 31 est configuré de telle sorte que le système de génération de champ magnétique 34 est décalé transversalement par rapport à la direction générale d'extension du chemin de roulement 9 lorsque la cuvette de réaction 2 est reçue dans le logement de réception 32. Plus particulièrement, le dispositif de détermination 31 est configuré de telle sorte que le système de génération de champ magnétique 34 est disposé au moins en partie en regard d'une paroi longitudinale, et par exemple la paroi longitudinale 6b, du réceptacle 3 lorsque la cuvette de réaction 2 est reçue dans le logement de réception 32.

Le système de génération de champ magnétique 34 comprend avantageusement deux électroaimants 34a, 34b disposés respectivement à proximité des extrémités du chemin de roulement 9 et d'un même côté du chemin de roulement 9, lorsque la cuvette de réaction 2 est reçue dans le logement de réception 32. Le champ magnétique généré par le système de génération de champ magnétique 34 peut être avantageusement réglé en faisant varier par exemple les écarts et/ou la longueur des impulsions électriques appliquées aux bobines des électroaimants 34a, 34b.

Le dispositif de détermination 31 comprend en outre un élément d'émission 35 configuré pour émettre un faisceau lumineux incident 36 en direction de l'échantillon sanguin à analyser 33 lorsque la cuvette de réaction 2 est reçue dans le logement de réception 32. L'élément d'émission 36 peut par exemple être une diode électroluminescente.

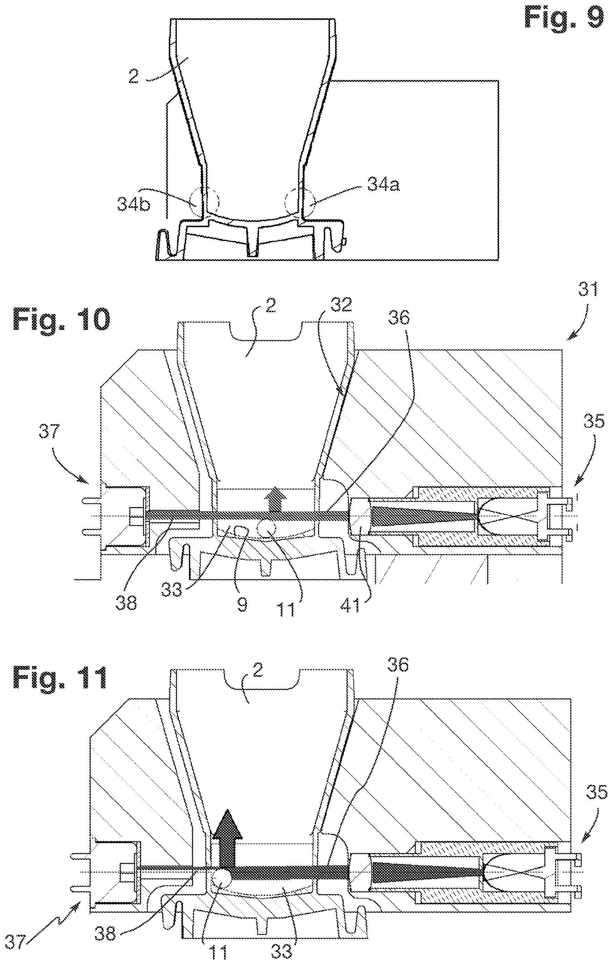

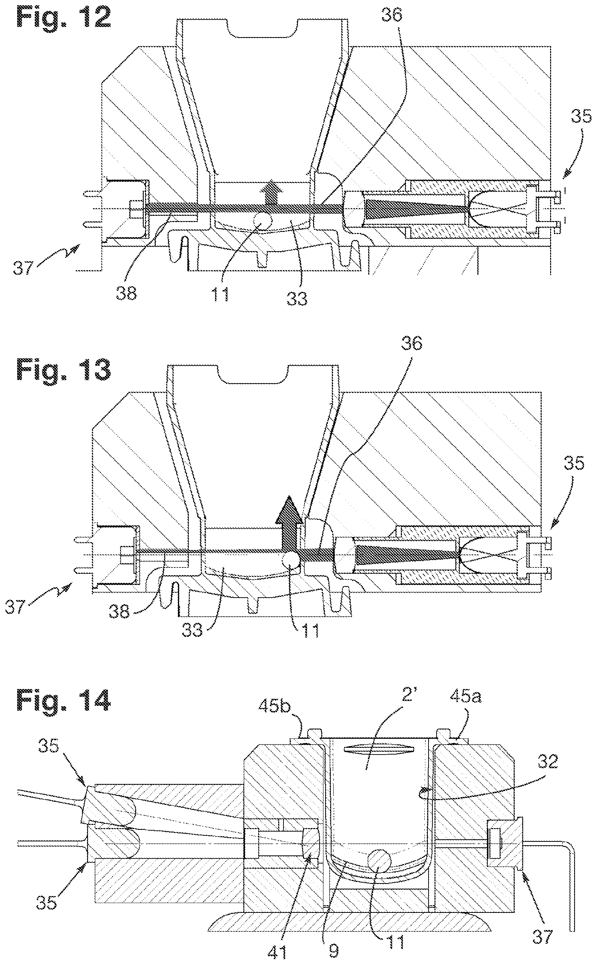

Selon le mode de réalisation représenté sur les figures, le dispositif de détermination 31 est configuré de telle sorte que, lorsque la cuvette de réaction 2 est reçue dans le logement de réception 32 et la bille ferromagnétique 11 se trouve au point le plus bas du chemin de roulement 9, la bille ferromagnétique 11 occulte en partie le faisceau lumineux incident 36 (voir les figures 10 et 12).

Selon un mode de réalisation de l'invention, le taux d'occultation du faisceau lumineux incident 36 varie entre une valeur minimale correspondant à une position de la bille ferromagnétique 11 au niveau du point le plus bas du chemin de roulement 9 (voir les figures 10 et 12) et une valeur maximale correspondant à une position de la bille ferromagnétique 11 la plus éloignée du point le plus bas du chemin de roulement 9 (voir les figures 11 et 13), la valeur minimal étant comprise par exemple entre 5 et 10%.

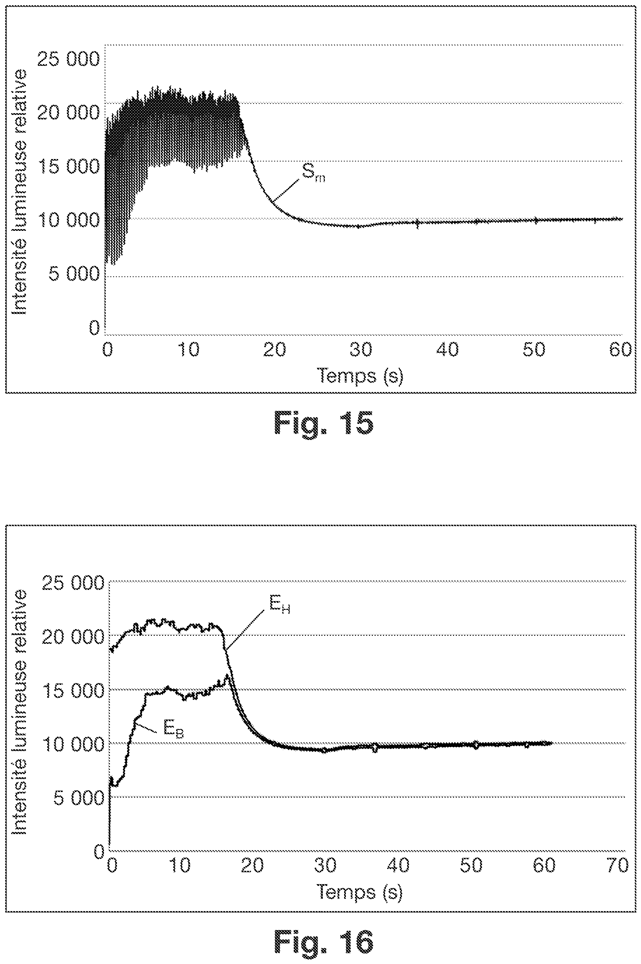

Le dispositif de détermination 31 comprend de plus un élément de détection 37 configuré pour détecter au moins un faisceau lumineux transmis 38 à travers la cuvette de réaction 2 et issu du faisceau lumineux incident 36, et pour fournir en sortie un signal de mesure S_{M}. La figure 15 représente l'évolution de l'amplitude d'un exemple de signal de mesure S_{M} en fonction du temps, et plus particulièrement l'évolution de l'intensité lumineuse relative d'un exemple de signal de mesure S_{M} en fonction du temps.

L'élément de détection 37 peut par exemple être un photodétecteur, tel qu'une photodiode. Selon le mode de réalisation représenté sur les figures, l'élément de détection 37 est situé sensiblement dans l'axe du faisceau lumineux incident 36. Ainsi, selon le mode de réalisation représenté sur les figures, les éléments d'émission et de détection sont disposés de part et d'autre des extrémités du chemin de roulement 9 lorsque la cuvette de réaction 2 est reçue dans le logement de réception 32.

Selon un mode de réalisation de l'invention, le signal de mesure S_{M} est obtenu par échantillonnage d'un signal continu à intervalles réguliers, la durée d'un intervalle, c'est-à-dire entre deux instants d'échantillonnage, étant par exemple de l'ordre de 10 ms.

Le dispositif de détermination 31 comprend également une unité de traitement 39. Comme cela est montré notamment sur la figure 5, l'unité de traitement 39 peut être disposée à proximité du logement de réception 32. Néanmoins, l'unité de traitement 39 pourrait également être déportée à distance de la zone de mesure.

L'unité de traitement 39 est plus particulièrement configurée pour :
- réaliser un premier traitement du signal de mesure S_{M} de façon à fournir un premier signal S1 représentatif de la variation d'au moins une grandeur physique représentative du mouvement de la bille ferromagnétique 11, l'au moins une grandeur physique représentative du mouvement de la bille ferromagnétique 11 étant par exemple l'amplitude et/ou de la fréquence du mouvement de la bille ferromagnétique 11,
- réaliser un deuxième traitement du signal de mesure S_{M} de façon à fournir un deuxième signal S2 représentatif de la variation d'au moins une propriété optique de l'échantillon sanguin à analyser 33, l'au moins une propriété optique de l'échantillon sanguin à analyser 33 étant par exemple l'absorbance de l'échantillon sanguin à analyser 33,
- déterminer une première valeur t1 du temps de coagulation de l'échantillon sanguin à analyser à partir du premier signal S1, et
- déterminer une deuxième valeur t2 du temps de coagulation de l'échantillon sanguin à analyser à partir du deuxième signal S2.

Selon un mode de réalisation de l'invention, l'unité de traitement 39 est configurée de telle sorte que le premier signal S1 fourni correspond à l'écart entre une enveloppe haute et une enveloppe basse du signal de mesure S_{M}. L'enveloppe haute du signal de mesure est déterminée en reliant les maximums locaux du signal de mesure S_{M}, tandis que l'enveloppe basse du signal de mesure est déterminée en reliant les minimums locaux du signal de mesure S_{M}. La figure 16 représente l'évolution de l'amplitude des enveloppes basse et haute EB et EH en fonction du temps, et plus particulièrement l'évolution de l'intensité lumineuse relative des enveloppes basse et haute EB et EH en fonction du temps.

Selon un autre mode de réalisation de l'invention, l'unité de traitement 39 est configurée de telle sorte que le premier signal S1 fourni correspond à une moyenne glissante de l'écart entre l'enveloppe haute et l'enveloppe basse du signal de mesure S_{M} sur un ensemble prédéterminé de valeurs de l'écart entre l'enveloppe haute et l'enveloppe basse du signal de mesure S_{M}, par exemple douze, correspondant à des instants de mesure ou d'échantillonnage successifs. De préférence, chaque valeur du premier signal S1 pour un instant de mesure ou d'échantillonnage donné est déterminée comme une moyenne glissante des dernières valeurs de l'écart entre l'enveloppe haute et l'enveloppe basse du signal de mesure S_{M}, par exemple des douze dernières valeurs de l'écart entre l'enveloppe haute et l'enveloppe basse du signal de mesure S_{M}.

Selon un mode de réalisation de l'invention, l'unité de traitement 39 est configurée pour fournir un signal de base correspondant à une moyenne glissante du premier signal S1 sur un intervalle de glissement prédéterminé. Plus particulièrement, chaque valeur du signal de base pour un instant de mesure ou d'échantillonnage donné est déterminée comme une moyenne glissante d'un ensemble de valeurs du premier signal S1 correspondant à des instants de mesure ou d'échantillonnage compris dans un intervalle de temps dont les bornes sont définies par référence à l'instant de mesure ou d'échantillonnage donné. Par exemple, l'intervalle de temps est de 10 secondes, et précède, pour chaque valeur du signal de base, l'instant de mesure ou d'échantillonnage donné respectif.

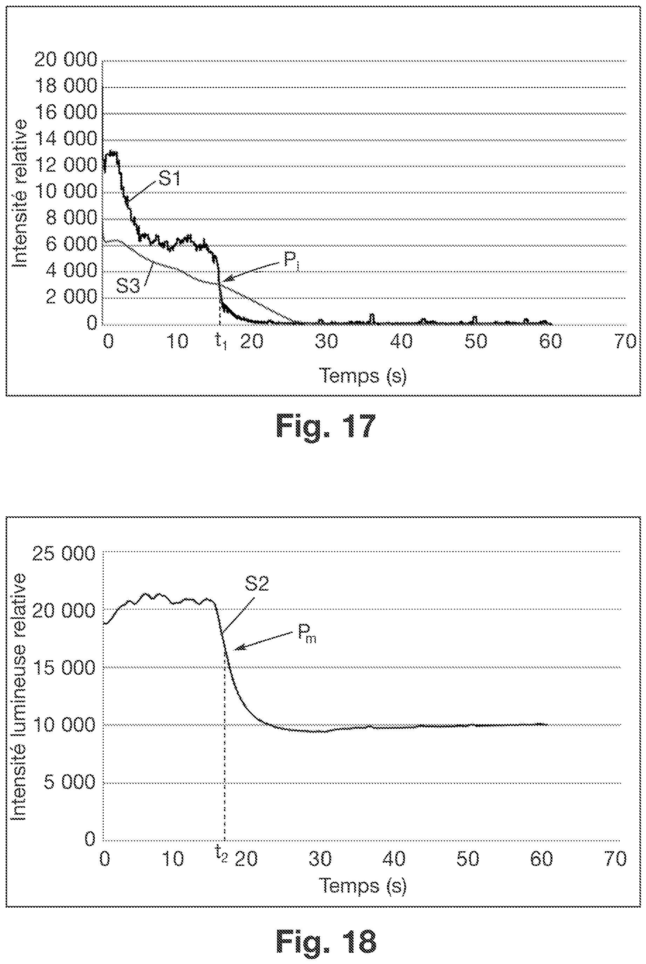

Selon un autre mode de mise en œuvre du procédé de détermination, chaque valeur du signal de base pour un instant de mesure ou d'échantillonnage donné est déterminée comme une moyenne glissante d'un ensemble de valeurs incluant la valeur du premier signal S1 à l'instant de mesure ou d'échantillonnage donné respectif et la totalité des valeurs du premier signal S1 correspondant à des instants de mesure ou d'échantillonnage précédant l'instant de mesure ou d'échantillonnage donné respectif. Selon un mode de réalisation de l'invention, l'unité de traitement 39 est configurée pour déterminer le point d'intersection Pi entre le premier signal S1 et un troisième signal S3 correspondant à un pourcentage prédéterminé du signal de base, la première valeur t1 du temps de coagulation de l'échantillon sanguin à analyser déterminé par l'unité de traitement 39 étant alors l'instant correspondant au point d'intersection Pi déterminé. Selon un mode de réalisation de l'invention, le troisième signal est compris entre 30 et 60% du signal de base. La figure 17 représente l'évolution de l'amplitude des premier et troisième signaux S1, S3 en fonction du temps, et plus particulièrement l'évolution de l'intensité lumineuse relative des premier et troisième signaux S1, S3 en fonction du temps. Il doit être noté que le pourcentage prédéterminé du signal de base peut être programmé en fonction des tests à réaliser, et par exemple en fonction du volume réactionnel des cuvettes de réaction utilisées.

Selon un mode de réalisation de l'invention, l'unité de traitement 39 est configurée de telle sorte que le deuxième signal S2 fourni correspond à une enveloppe haute moyennée du signal de mesure SM. Par exemple, l'unité de traitement 39 est configurée de telle sorte que le deuxième signal S2 fourni correspond à une moyenne glissante de l'enveloppe haute du signal de mesure sur un ensemble prédéterminé de valeurs de l'enveloppe haute, par exemple douze, correspondant à des instants de mesure ou d'échantillonnage successifs. De préférence, chaque valeur du deuxième signal S2 pour un instant de mesure ou d'échantillonnage donné est déterminée comme une moyenne glissante des dernières valeurs de l'enveloppe haute, par exemple des douze dernières valeurs de l'enveloppe haute.

La figure 18 représente l'évolution de l'amplitude de l'enveloppe haute moyennée en fonction du temps, et plus particulièrement l'évolution de l'intensité lumineuse relative de l'enveloppe haute moyennée en fonction du temps.

Selon un mode de réalisation de l'invention, l'unité de traitement 39 est configurée pour déterminer la pente maximale Pm du deuxième signal S2, la deuxième valeur t2 du temps de coagulation de l'échantillon sanguin à analyser déterminé par l'unité de traitement 39 étant alors l'instant correspondant à ladite pente maximale.

Selon un mode de réalisation de l'invention, le dispositif de détermination 31 comprend une lentille optique 41 disposée sur le trajet du faisceau lumineux incident et configurée pour collimater le faisceau lumineux incident 36.

Le dispositif de détermination 31 comprend en outre un système de chargement 42 configuré pour charger et décharger des cuvettes de réaction 2 dans et hors du logement de réaction. Le système de chargement 42 comporte avantageusement un actionneur linéaire, qui peut par exemple comprendre un moteur électrique 43, tel qu'un moteur électrique pas à pas.

Le dispositif de détermination 31 comprend en outre avantageusement un premier et un deuxième éléments ou corps 44a, 44b délimitant respectivement une première et une deuxième portions de logement 32a, 32b. Les premier et deuxième éléments 44a, 44b sont montés mobiles l'un par rapport à l'autre entre une position de chargement ou de déchargement (voir la figure 5) dans laquelle les premier et deuxième éléments 44a, 44b sont éloignés l'un de l'autre et autorisent un déplacement de la cuvette de réaction 2 jusqu'en regard des première et deuxième portions de logement 32a, 32b, et une position de mesure dans laquelle les premier et deuxième éléments 44a, 44b sont rapprochés l'un de l'autre et délimitent le logement de réception 32.

Un procédé de détermination du temps de coagulation d'un échantillon sanguin à analyser à l'aide du dispositif de détermination 31 va maintenant être décrit.

Un tel procédé de détermination comprend les étapes suivantes consistant à :
- prévoir une cuvette de réaction 2 contenant l'échantillon sanguin à analyser 33,
- placer une bille ferromagnétique 11 sur le chemin de roulement 9 de la cuvette de réaction 2,
- placer la cuvette de réaction 2 dans le logement de réception 32 du dispositif de détermination 31,
- générer un champ magnétique à l'aide du système de génération de champ magnétique 34 de manière à déplacer la bille ferromagnétique 11 le long du chemin de roulement 9 selon mouvement oscillatoire, le champ magnétique étant généré en alimentant séquentiellement les bobines des deux électroaimants 34a, 34b l'une après l'autre,
- émettre un faisceau lumineux incident 35 en direction de l'échantillon sanguin à analyser 33 à l'aide de l'élément d'émission 36,
- détecter, par exemple toutes les 20 ms, un faisceau lumineux 38 transmis à travers la cuvette de réaction 2 et issu du faisceau lumineux incident 36 à l'aide de l'élément de détection 37 de façon à fournir un signal de mesure S_{M},
- réaliser un premier traitement du signal de mesure S_{M} à l'aide de l'unité de traitement 39 de façon à fournir un premier signal S1 représentatif de la variation notamment de l'amplitude du mouvement de la bille ferromagnétique 11,
- réaliser un deuxième traitement du signal de mesure S_{M} à l'aide de l'unité de traitement 39 de façon à fournir un deuxième signal S2 représentatif de la variation notamment de l'absorbance de l'échantillon sanguin à analyser 33,
- déterminer à l'aide de l'unité de traitement 39 une première valeur t1 du temps de coagulation de l'échantillon sanguin à analyser à partir du premier signal S1,
- déterminer à l'aide de l'unité de traitement 39 une deuxième valeur t2 du temps de coagulation de l'échantillon sanguin à analyser à partir du deuxième signal S2, et
- comparer les première et deuxième valeurs t1, t2 déterminées du temps de coagulation.

Selon un mode de mise en œuvre du procédé de détermination, ce dernier comprend en outre une étape consistant à régler, et plus précisément à asservir, l'intensité lumineuse du faisceau lumineux incident 36 en fonction d'une valeur initiale du signal de mesure S_{M} correspondant à une position de la bille ferromagnétique 11 sensiblement au niveau du point le plus bas du chemin de roulement 9. Ces dispositions permettent d'asservir l'intensité lumineuse du faisceau lumineux incident 36 sur l'absorbance initiale de l'échantillon sanguin à analyser, et par exemple à augmenter ladite intensité lumineuse si l'échantillon sanguin à analyser est initialement très absorbant, ou inversement, et ce afin notamment d'avoir un signal de référence le plus élevé possible, sans risque de saturation.

Selon un mode de mise en œuvre du procédé de détermination, ce dernier comprend en outre une étape consistant à régler, et plus précisément à asservir, durant une phase initiale du procédé de détermination, par exemple de l'ordre de 1 à 2 secondes à partir du déclenchement du mouvement de la bille ferromagnétique, au moins un paramètre représentatif du champ magnétique auquel est soumise la bille ferromagnétique 11 en fonction des valeurs initiales du signal de mesure S_{M}, et plus particulièrement en fonction des valeurs initiales du premier signal S1. L'au moins un paramètre représentatif du champ magnétique peut par exemple être la période et/ou l'intensité du champ magnétique généré par le système de génération de champ magnétique 34. Ces dispositions permettent d'optimiser l'oscillation de la bille ferromagnétique 11 en fonction de la viscosité initiale de l'échantillon sanguin à analyser 33, et donc par exemple d'éviter des chocs de la bille ferromagnétique 11 contre les parois de la cuvette de réaction 2, ou, à l'inverse, d'éviter que l'amplitude maximale de la bille ferromagnétique 11 soit insuffisante.

La figure 14 représente un dispositif de détermination 31 selon un deuxième mode de réalisation de l'invention qui diffère de celui représenté sur les figures 5 à 13 essentiellement en ce que le système de chargement 42 et le logement de réception 2 sont adaptés respectivement pour charger et recevoir un ensemble ou bloc comprenant une pluralité de cuvettes de réaction 2' reliées les unes aux autres. Les différentes cuvettes de réaction 2' d'un tel ensemble ou bloc sont par exemple moulées d'un seul tenant en une matière plastique. Les cuvettes de réaction 2' sont avantageusement disposées de manière adjacente de telle sorte que leurs parois longitudinales soient parallèles les unes autre autres. Les cuvettes de réaction 2' sont par exemple reliées entres elles au niveau de leur partie supérieure par des portions de liaison latérales 45a, 45b.

Comme il va de soi, l'invention ne se limite pas aux seules formes d'exécution de ce dispositif de détermination et de cette cuvette de réaction, décrites ci-dessus à titre d'exemples, elle en embrasse au contraire toutes les variantes de réalisation, en étant limitée seulement par la portée des revendications.

## Revendications

1. Procédé de détermination du temps de coagulation d'un échantillon sanguin à analyser, comprenant les étapes suivantes :
- prévoir une cuvette de réaction (2) contenant l'échantillon sanguin à analyser (33), la cuvette de réaction (2) comprenant un fond (8) délimitant un chemin de roulement (9) concave dont la concavité est dirigée vers le haut,
- placer une bille ferromagnétique (11) sur le chemin de roulement (9) de la cuvette de réaction (2),
- soumettre la bille ferromagnétique (11) à un champ magnétique de manière à déplacer la bille ferromagnétique le long du chemin de roulement (9) selon mouvement oscillatoire,
- exposer l'échantillon sanguin à analyser à un faisceau lumineux incident (36) configuré pour être au moins en partie occulté par la bille ferromagnétique (11) lors d'au moins une partie du mouvement oscillatoire de la bille ferromagnétique le long du chemin de roulement (9),
- détecter au moins un faisceau lumineux (38) transmis à travers la cuvette de réaction (2) et issu du faisceau lumineux incident (36) de façon à fournir un signal de mesure (S_{M}),
**caractérisé en ce qu'**il comprend:
- réaliser un premier traitement du signal de mesure (S_{M}) de façon à fournir un premier signal (S1) représentatif de la variation d'au moins une grandeur physique représentative du mouvement de la bille ferromagnétique (11),
- réaliser un deuxième traitement du signal de mesure (S_{M}) de façon à fournir un deuxième signal (S2) représentatif de la variation d'au moins une propriété optique de l'échantillon sanguin à analyser,
- déterminer une première valeur (t1) du temps de coagulation de l'échantillon sanguin à analyser à partir du premier signal, et
- déterminer une deuxième valeur (t2) du temps de coagulation de l'échantillon sanguin à analyser à partir du deuxième signal.

2. Procédé de détermination selon la revendication 1, dans lequel le premier traitement du signal de mesure (S_{M}) est réalisé de telle sorte que le premier signal (S1) fourni correspond à l'écart entre une enveloppe haute et une enveloppe basse du signal de mesure (S_{M}).

3. Procédé de détermination selon la revendication 1 ou 2, dans lequel l'étape de détermination de la première valeur (t1) du temps de coagulation de l'échantillon sanguin à analyser comprend une étape consistant à fournir un signal de base correspondant à une moyenne glissante du premier signal (S1), la première valeur du temps de coagulation de l'échantillon sanguin à analyser étant déterminée à partir du signal de base.

4. Procédé de détermination selon l'une quelconque des revendications 1 à 3, dans lequel le deuxième traitement du signal de mesure (S_{M}) est réalisé de telle sorte que le deuxième signal (S2) fourni correspond à une moyenne glissante de l'enveloppe haute du signal de mesure.

5. Procédé de détermination selon la revendication 4, dans lequel l'étape de détermination de la deuxième valeur (t2) du temps de coagulation de l'échantillon sanguin à analyser comprend une étape consistant à déterminer la pente maximale du deuxième signal (S2), la deuxième valeur (t2) du temps de coagulation de l'échantillon sanguin à analyser étant l'instant correspondant à ladite pente maximale.

6. Procédé de détermination selon l'une quelconque des revendications 1 à 5, lequel comprend une étape consistant à comparer les première et deuxième valeurs (t1, t2) du temps de coagulation déterminées.

7. Procédé de détermination selon l'une quelconque des revendications 1 à 6, lequel comprend en outre une étape consistant à régler l'intensité lumineuse du faisceau lumineux incident (36) en fonction d'une valeur initiale du signal de mesure.

8. Procédé de détermination selon l'une quelconque des revendications 1 à 7, lequel comprend en outre une étape consistant à régler, durant une phase initiale du procédé de détermination, au moins un paramètre représentatif du champ magnétique auquel est soumise la bille ferromagnétique (11) en fonction des valeurs initiales du signal de mesure (S_{M}).

9. Dispositif de détermination (31) du temps de coagulation d'un échantillon sanguin à analyser, le dispositif de détermination comprenant :
- un logement de réception (32) dans lequel est destinée à être reçue une cuvette de réaction (2) contenant l'échantillon sanguin à analyser (33) et une bille ferromagnétique (11), la cuvette de réaction (2) délimitant un chemin de roulement (9) concave dont la concavité est dirigée vers le haut et sur lequel est placé la bille ferromagnétique (11),
- un système de génération de champ magnétique (34) configuré pour générer un champ magnétique apte à déplacer la bille ferromagnétique (11) le long du chemin de roulement (9) selon mouvement oscillatoire lorsque la cuvette de réaction (2) est reçue dans le logement de réception,
- un élément d'émission (35) configuré pour émettre un faisceau lumineux incident (36) en direction de l'échantillon sanguin à analyser (33) lorsque la cuvette de réaction (2) est reçue dans le logement de réception (32), le faisceau lumineux incident (36) étant configuré pour être au moins en partie occulté par la bille ferromagnétique (11) lors d'au moins une partie du mouvement de la bille ferromagnétique le long du chemin de roulement (9),
- un élément de détection (37) configuré pour détecter au moins un faisceau lumineux (38) transmis à travers la cuvette de réaction (2) et issu du faisceau lumineux incident (36) et pour fournir en sortie un signal de mesure (S_{M}), et
- une unité de traitement (39) configurée pour :
- réaliser un premier traitement du signal de mesure (S_{M}) de façon à fournir un premier signal (S1) représentatif de la variation d'au moins une grandeur physique représentative du mouvement de la bille ferromagnétique (11),
- réaliser un deuxième traitement du signal de mesure (S_{M}) de façon à fournir un deuxième signal représentatif de la variation d'au moins une propriété optique de l'échantillon sanguin à analyser,
- déterminer une première valeur (t1) du temps de coagulation de l'échantillon sanguin à analyser à partir du premier signal, et
- déterminer une deuxième valeur (t2) du temps de coagulation de l'échantillon sanguin à analyser à partir du deuxième signal.

10. Dispositif de détermination (31) selon la revendication 9, dans lequel le système de génération de champ magnétique (34) est décalé transversalement par rapport à la direction générale d'extension du chemin de roulement (9).

11. Dispositif de détermination (31) selon la revendication 9 ou 10, lequel est configuré de telle sorte que, lorsque la cuvette de réaction (2) est reçue dans le logement de réception (32) et la bille ferromagnétique (11) se trouve au point le plus bas du chemin de roulement (9), la bille ferromagnétique (11) occulte en partie le faisceau lumineux incident (36).

12. Cuvette de réaction (2) adaptée au dispositif de détermination (31) selon l'une des revendications 9 à 11, tel que le dispositif de détermination (31) et la cuvette de réaction (2) permettent la mise en œuvre du procédé selon l'une des revendications 1 à 8, la cuvette de réaction (2) comprenant :
- un réceptacle (3) configuré pour contenir un fluide biologique à analyser, le réceptacle (3) comprenant :
- une partie inférieure (4) comportant un fond (8) délimitant un chemin de roulement (9) concave dont la concavité est dirigée vers le haut, le chemin de roulement (9) présentant son point le plus bas sensiblement en son centre,
- une partie supérieure (5) délimitant une ouverture d'introduction (14),
- des premiers moyens d'accrochage configurés pour accrocher la cuvette de réaction (2) à une première cuvette de réaction adjacente selon une première direction d'accrochage (D1), et
- des deuxième moyens d'accrochage configurés pour accrocher la cuvette de réaction (2) à une deuxième cuvette de réaction adjacente selon une seconde direction d'accrochage (D2) qui est sensiblement perpendiculaire à la première direction d'accrochage (D1),
la cuvette de réaction (2) contenant une bille ferromagnétique (11) placée sur le chemin de roulement (9), le chemin de roulement (9) étant destiné à guider un mouvement oscillatoire de la bille ferromagnétique (11), la bille ferromagnétique (11) étant configurée pour occulter au moins en partie un faisceau lumineux incident (36) émit par l'élément d'émission (35) du dispositif de détermination (31) lors d'au moins une partie du mouvement de la bille ferromagnétique (11) le long du chemin de roulement (9) et lorsque la cuvette de réaction (2) est reçue dans le logement de réception (32) du dispositif de détermination,
la cuvette de réaction (2) étant configurée de telle sorte que l'élément d'émission (35) et l'élément de détection (37) sont disposés sensiblement dans l'axe du chemin de roulement (9) lorsque la cuvette de réaction (2) est reçue dans le logement de réception (32) du dispositif de détermination (31),
la cuvette de réaction (2) étant **caractérisée en ce que** la largeur de la partie inférieure (4) du réceptacle (3) transversalement à la direction générale d'extension du chemin de roulement (9) est inférieure la largeur de la partie supérieure (5) du réceptacle (3) transversalement à la direction d'extension du chemin de roulement (9), et **en ce que** le chemin de roulement (9) est décalé transversalement par rapport à un plan longitudinal médian (P1) de la partie supérieure (5) du réceptacle (3).

## Patentansprüche

1. Verfahren zur Bestimmung der Koagulationszeit einer zu analysierenden Blutprobe, die folgenden Schritte umfassend:
- Vorsehen eines Reaktionsgefäßes (2), die zu analysierende Blutprobe (33) enthaltend, wobei das Reaktionsgefäß (2) eine Rückwand (8) umfasst, eine konkave Kranbahn (9) eingrenzend, deren Konkavität nach oben gerichtet ist,
- Platzieren eines ferromagnetischen Lagers (11) auf der Kranbahn (9) des Reaktionsgefäßes (2),
- Unterziehen des ferromagnetischen Lagers (11) einem magnetischen Feld, solcherart, dass das ferromagnetische Lager entlang der Kranbahn (9) gemäß oszillierender Bewegung deplatziert wird,
- Aussetzen der zu analysierenden Blutprobe einem einfallenden Lichtstrahl (36), konfiguriert, um während wenigstens eines Teils der oszillierenden Bewegung des elektromagnetischen Lagers entlang der Kranbahn (9) wenigstens teilweise von dem ferromagnetischen Lager (11) verdunkelt zu werden,
- Detektieren wenigstens eines Lichtstrahls (38), über das Reaktionsgefäß (2) übermittelt und von dem einfallenden Lichtstrahl (36) stammend, sodass ein Messsignal (S_{M}) bereitgestellt wird,
**dadurch gekennzeichnet, dass** es Folgendes umfasst:
- Durchführen einer ersten Behandlung des Messsignals (S_{M}), sodass ein erstes Signal (S1) bereitgestellt wird, repräsentativ für die Variation wenigstens einer physikalischen Größe, repräsentativ für die Bewegung des ferromagnetischen Lagers (11),
- Durchführen einer zweiten Behandlung des Messsignals (S_{M}), sodass ein zweites Signal (S2) bereitgestellt wird, repräsentativ für die Variation wenigstens einer optischen Eigenschaft der zu analysierenden Blutprobe,
- Bestimmen eines ersten Wertes (t1) der Koagulationszeit der zu analysierenden Blutprobe von dem ersten Signal aus, und
- Bestimmen eines zweiten Wertes (t2) der Koagulationszeit der zu analysierenden Blutprobe von dem zweiten Signal aus.

2. Bestimmungsverfahren nach Anspruch 1, in welchem die erste Behandlung des Messsignals (S_{M}) solcherart durchgeführt wird, dass das erste bereitgestellte Signal (S1) der Abweichung zwischen einer hohen Hüllkurve und einer niedrigen Hüllkurve des Messsignals (S_{M}) entspricht.

3. Bestimmungsverfahren nach Anspruch 1 oder 2, in welchem der Schritt des Bestimmens des ersten Wertes (t1) der Koagulationszeit der zu analysierenden Blutprobe einen Schritt umfasst bestehend in dem Bereitstellen eines Basissignals, einem gleitenden Mittelwert des ersten Signals (S1) entsprechend, wobei der erste Wert der Koagulationszeit der zu analysierenden Blutprobe von dem Basissignal aus bestimmt wird.

4. Bestimmungsverfahren nach einem der Ansprüche 1 bis 3, in welchem die zweite Behandlung des Messsignals (S_{M}) solcherart durchgeführt wird, dass das bereitgestellte Signal (S2) einem gleitenden Mittelwert der hohen Umhüllungskurve des Messsignals entspricht.

5. Bestimmungsverfahren nach Anspruch 4, in welchem der Schritt der Bestimmung des zweiten Wertes (t2) der Koagulationszeit der zu analysierenden Blutprobe einen Schritt umfasst bestehend in dem Bestimmen der maximalen Steigung des zweiten Signals (S2), wobei es sich bei dem zweiten Wert (t2) der Koagulationszeit der zu analysierenden Blutprobe um den der maximalen Steigung entsprechenden Moment handelt.

6. Bestimmungsverfahren nach einem der Ansprüche 1 bis 5, welches einen Schritt umfasst bestehend in dem Vergleichen der bestimmten ersten und zweiten Werte (t1, t2) der Koagulationszeit.

7. Bestimmungsverfahren nach einem der Ansprüche 1 bis 6, welches weiter einen Schritt umfasst bestehend aus dem Regeln der Lichtintensität des einfallenden Lichtstrahls (36) gemäß einem Anfangswert des Messsignals.

8. Bestimmungsverfahren nach einem der Ansprüche 1 bis 7, welches weiter einen Schritt des Regelns von wenigstens einem Parameter, repräsentativ für das elektromagnetische Feld, welchem das ferromagnetische Lager (11) unterzogen wird, gemäß der Anfangswerte des Messsignals (S_{M}) während einer Anfangsphase des Bestimmungsprozesses umfasst.

9. Bestimmungsvorrichtung (31) für die Koagulationszeit einer zu analysierenden Blutprobe, wobei die Bestimmungsvorrichtung Folgendes umfasst:
- ein Aufnahmegehäuse (32), wobei ein Reaktionsgefäß (2), die zu analysierende Blutprobe (33) umfassend, dazu bestimmt ist, in diesem aufgenommen zu werden, und ein ferromagnetisches Lager (11), wobei das Reaktionsgefäß (2) eine konkave Kranbahn (9) begrenzt, deren Konkavität nach oben gerichtet ist und auf welcher das ferromagnetische Lager (11) platziert ist,
- ein Erzeugungssystem für magnetische Felder (34), konfiguriert, um ein magnetisches Feld zu erzeugen, geeignet, um das ferromagnetische Lager (11) entlang der Kranbahn (9) gemäß oszillierender Bewegung zu deplatzieren, während das Reaktionsgefäß (2) in dem Aufnahmegehäuse aufgenommen ist,
- ein Sendeelement (35), konfiguriert, um einen einfallenden Lichtstrahl (36) in Richtung der zu analysierenden Blutprobe (33) auszusenden, wenn das Reaktionsgefäß (2) in dem Aufnahmegehäuse (32) aufgenommen ist, wobei der einfallende Lichtstrahl (36) konfiguriert ist, um während wenigstens eines Teils der Bewegung des ferromagnetischen Lagers entlang der Kranbahn (9) wenigstens teilweise von dem ferromagnetischen Lager (11) verdunkelt zu werden,
- ein Detektionselement (37), konfiguriert, um wenigstens einen Lichtstrahl (38) zu detektieren, übermittelt über das Reaktionsgefäß (2) und stammend von dem einfallenden Lichtstrahl (36), und um ein Messsignal (S_{M}) auszugeben, und
- eine Behandlungseinheit (39), zu Folgendem konfiguriert:
- Durchführen einer ersten Behandlung des Messsignals (S_{M}), sodass ein erstes Signal (S1) bereitgestellt wird, repräsentativ für die Variation wenigstens einer physikalischen Größe, repräsentativ für die Bewegung des ferromagnetischen Lagers (11),
- Durchführen einer zweiten Behandlung des Messsignals (S_{M}), sodass ein zweites Signal bereitgestellt wird, repräsentativ für die Variation wenigstens einer optischen Eigenschaft der zu analysierenden Blutprobe,
- Bestimmen eines ersten Wertes (t1) der Koagulationszeit der zu analysierenden Blutprobe von dem ersten Signal aus, und
- Bestimmen eines zweiten Wertes (t2) der Koagulationszeit der zu analysierenden Blutprobe von dem zweiten Signal aus.

10. Bestimmungsvorrichtung (31) nach Anspruch 9, in welcher das System zum Erzeugen des magnetischen Feldes (34) in Bezug auf die allgemeine Erstreckungsrichtung der Kranbahn (9) transversal versetzt ist.

11. Bestimmungsverfahren (31) nach Anspruch 9 oder 10, welches solcherart konfiguriert ist, dass das elektromagnetische Lager (11) teilweise den einfallenden Lichtstrahl (36) verdunkelt, während das Reaktionsgefäß (2) in dem Aufnahmegehäuse (32) aufgenommen ist und sich das elektromagnetische Lager (11) am tiefsten Punkt der Kranbahn (9) befindet.

12. Reaktionsgefäß (2), angepasst an die Bestimmungsvorrichtung (31) nach einem der Ansprüche 9 bis 11, sodass die Bestimmungsvorrichtung (31) und das Reaktionsgefäß (2) die Durchführung des Verfahrens nach einem der Ansprüche 1 bis 8 ermöglichen, wobei das Reaktionsgefäß (2) Folgendes umfasst:
- einen Behälter (3), konfiguriert, um ein zu analysierendes biologisches Fluid zu enthalten, wobei der Behälter (3) Folgendes umfasst:
- einen unteren Teil (4), umfassend eine Rückwand (8), eine konkave Kranbahn (9) begrenzend, deren Konkavität nach oben gerichtet ist, wobei die Kranbahn (9) ihren untersten Punkt im Wesentlichen in ihrem Zentrum aufweist,
- einen oberen Teil (5), eine Einführungsöffnung (14) begrenzend,
- erste Befestigungsmittel, konfiguriert, um das Reaktionsgefäß (2) entsprechend einer ersten Befestigungsrichtung (D1) an einem ersten benachbarten Reaktionsgefäß zu befestigen, und
- zweite Befestigungsmittel, konfiguriert, um das Reaktionsgefäß (2) entsprechend einer zweiten Befestigungsrichtung (D2), welche im Wesentlichen senkrecht zu der ersten Befestigungsrichtung (D1) ist, an einem zweiten benachbarten Reaktionsgefäß zu befestigen,
wobei das Reaktionsgefäß (2) ein auf der Kranbahn (9) platziertes ferromagnetisches Lager (11) enthält, wobei die Kranbahn (9) dafür bestimmt ist, eine oszillatorische Bewegung des ferromagnetischen Lagers (11) zu führen, wobei das ferromagnetische Lager (11) konfiguriert ist, um während wenigstens eines Teils der Bewegung des ferromagnetischen Lagers (11) entlang der Kranbahn (9) und während das Reaktionsgefäß (2) in dem Aufnahmegehäuse (32) der Bestimmungsvorrichtung aufgenommen ist einen von dem Sendeelement (35) der Bestimmungsvorrichtung (31) ausgesendeten einfallenden Lichtstrahl (36) wenigstens teilweise zu verdunkeln,
wobei das Reaktionsgefäß (2) solcherart konfiguriert ist, dass das Sendeelement (35) und das Detektionselement (37) im Wesentlichen in der Achse der Kranbahn (9) angeordnet ist, während das Reaktionsgefäß (2) in dem Aufnahmegehäuse (32) der Bestimmungsvorrichtung (31) aufgenommen ist,
wobei das Reaktionsgefäß (2) **dadurch gekennzeichnet ist, dass** die Breite des unteren Teils (4) des Behälters (3) quer zu der allgemeinen Erstreckungsrichtung der Kranbahn (9) geringer ist als die Breite des oberen Teils (5) des Behälters (3) quer zu der Erstreckungsrichtung der Kranbahn (9), und dadurch, dass die Kranbahn (9) in Bezug auf eine längsseitige Medianebene (P1) des oberen Teils (5) des Gefäßes (3) versetzt ist.

## Claims

1. A method for determining the coagulation time of a blood sample to be analyzed, comprising the following steps:
- providing a reaction cuvette (2) containing the blood sample to be analyzed (33), the reaction cuvette (2) comprising a bottom (8) delimiting a concave raceway (9) whose concavity is directed upwards,
- placing a ferromagnetic ball (11) on the raceway (9) of the reaction cuvette (2),
- subjecting the ferromagnetic ball (11) to a magnetic field so as to displace the ferromagnetic ball along the raceway (9) in an oscillatory movement,
- exposing the blood sample to be analyzed to an incident light beam (36) configured to be at least partially obscured by the ferromagnetic ball (11) during at least one part of the oscillatory movement of the ferromagnetic ball along the raceway (9),
- detecting at least one light beam (38) transmitted through the reaction cuvette (2) and coming from the incident light beam (36) so as to provide a measurement signal (S_{M}),
**characterized in that** it comprises:
- carrying out a first processing of the measurement signal (S_{M}) so as to provide a first signal (S1) representative of the variation of at least one physical quantity representative of the movement of the ferromagnetic ball (11),
- carrying out a second processing of the measurement signal (S_{M}) so as to provide a second signal (S2) representative of the variation of at least one optical property of the blood sample to be analyzed,
- determining a first value (t1) of the coagulation time of the blood sample to be analyzed from the first signal, and
- determining a second value (t2) of the coagulation time of the blood sample to be analyzed from the second signal.

2. The determination method according to claim 1, wherein the first processing of the measurement signal (S_{M}) is carried out such that the provided first signal (S1) corresponds to the deviation between a high envelope and a low envelope of the measurement signal (S_{M}).

3. The determination method according to claim 1 or 2, wherein the step for determining the first value (t1) of the coagulation time of the blood sample to be analyzed comprises a step consisting in providing a base signal corresponding to a sliding average of the first signal (S1), the first value of the coagulation time of the blood sample to be analyzed being determined from the base signal.

4. A determination method according to any one of claims 1 to 3, wherein the second processing of the measurement signal (S_{M}) is carried out such that the provided second signal (S2) corresponds to a sliding average of the high envelope of the measurement signal.

5. The determination method according to claim 4, wherein the step for determining the second value (t2) of the coagulation time of the blood sample to be analyzed comprises a step consisting in determining the maximum slope of the second signal (S2), the second value (t2) of the coagulation time of the blood sample to be analyzed being the moment corresponding to said maximum slope.

6. The determination method according to any one of claims 1 to 5, which comprises a step consisting in comparing the first and second determined values (t1, t2) of the coagulation time.

7. The determination method according to any one of claims 1 to 6, which further comprises a step consisting in adjusting the light intensity of the incident light beam (36) depending on an initial value of the measurement signal.

8. The determination method according to any one of claims 1 to 7, which further comprises a step consisting in adjusting, during an initial phase of the determination method, at least one parameter representative of the magnetic field to which the ferromagnetic ball (11) is subjected depending on the initial values of the measurement signal (S_{M}).

9. A device for determining (31) the coagulation time of a blood sample to be analyzed, the determining device comprising:
- a receiving housing (32) in which a reaction cuvette (2) containing the blood sample to be analyzed (33) and a ferromagnetic ball (11) is intended to be received, the reaction cuvette (2) delimiting a concave raceway (9) whose concavity is directed upwards and on which the ferromagnetic ball (11) is placed,
- a magnetic field generation system (34) configured to generate a magnetic field capable of displacing the ferromagnetic ball (11) along the raceway (9) in an oscillatory movement when the reaction cuvette (2) is received in the receiving housing,
- an emission member (35) configured to emit an incident light beam (36) in the direction of the blood sample to be analyzed (33) when the reaction cuvette (2) is received in the receiving housing (32), the incident light beam (36) being configured to be at least partially obscured by the ferromagnetic ball (11) during at least one part of the movement of the ferromagnetic ball along the raceway (9),
- a detection member (37) configured to detect at least one light beam (38) transmitted through the reaction cuvette (2) and coming from the incident light beam (36) and to output a measurement signal (S_{M}), and
- a processing unit (39) configured:
• to carry out a first processing of the measurement signal (S_{M}) so as to provide a first signal (S1) representative of the variation of at least one physical quantity representative of the movement of the ferromagnetic ball (11),
• to carry out a second processing of the measurement signal (S_{M}) so as to provide a second signal representative of the variation of at least one optical property of the blood sample to be analyzed,
• to determine a first value (t1) of the coagulation time of the blood sample to be analyzed from the first signal, and
• to determine a second value (t2) of the coagulation time of the blood sample to be analyzed from the second signal.

10. The determination device (31) according to claim 9, wherein the magnetic field generation system (34) is transversely shifted relative to the general direction of extension of the raceway (9).

11. The determination device (31) according to claim 9 or 10, which is configured such that, when the reaction cuvette (2) is received in the receiving housing (32) and the ferromagnetic ball (11) is located at the lowest point of the raceway (9), the ferromagnetic ball (11) partially obscures the incident light beam (36).

12. A reaction cuvette (2) adapted to the determination device (31) according to one of claims 9 to 11, such that the determination device (31) and the reaction cuvette (2) allow the implementation of the method according to any of claims 1 to 8, the reaction cuvette (2) comprising:
- a receptacle (3) configured to contain a biological fluid to be analyzed, the receptacle (3) comprising:
• a lower portion (4) including a bottom (8) delimiting a concave raceway (9) whose concavity is directed upwards, the raceway (9) having the lowest point thereof substantially at its center,
• an upper portion (5) delimiting an insertion opening (14),
- first hooking means configured to hook the reaction cuvette (2) to a first adjacent reaction cuvette in a first hooking direction (D1), and
- second hooking means configured to hook the reaction cuvette (2) to a second adjacent reaction cuvette in a second hooking direction (D2) which is substantially perpendicular to the first hooking direction (D1),
the reaction cuvette (2) containing a ferromagnetic ball (11) placed on the raceway (9), the raceway (9) being intended to guide an oscillatory movement of the ferromagnetic ball (11), the ferromagnetic ball (11) being configured to partially obscure an incident light beam (36) emitted by the emission member (35) of the determination device (31) during at least one part of the movement of the ferromagnetic ball (11) along the raceway (9) and when the reaction cuvette (2) is received in the receiving housing (32) of the determination device (31),
the reaction cuvette (2) being characterized such that the emission member (35) and the detection member (37) are substantially disposed in the axis of the raceway (9) when the reaction cuvette (2) is received in the receiving housing (32) of the determination device (31),
the reaction cuvette (2) being **characterized in that** the width of the lower portion (4) of the receptacle (3) transversely to the general direction of extension of the raceway (9) is smaller than the width of the upper portion (5) of the receptacle (3) transversely to the direction of extension of the raceway (9), and **in that** the raceway (9) is transversely shifted relative to a median longitudinal plane (P1) of the upper portion (5) of the receptacle (3).
